# EUROPEAN PATENT SPECIFICATION

(11) **EP 4 279 082 B1**
(45) Date of publication and mention of the grant of the patent: **04.03.2026**
(21) Application number: 23187615.2
(22) Date of filing: 16.01.2020
(51) Int. Cl.: A61K 9/00, A61K 47/12, A61K 47/26, A61K 38/00, A61K 38/17

(54) **FORMULATIONS**
FORMULIERUNGEN
FORMULATIONS

(30) Priority: 17.01.2019 GB 201900658; 10.04.2019 GB 201905105
(43) Date of publication of application: 22.11.2023
(62) Divisional of application: 20701703.9
(73) Proprietor: Immunocore Limited, Abingdon, Oxfordshire OX14 4RY (GB)
(72) Inventor: JOHNSON, Andy, Abingdon, OX14 4RY (GB); EBNER, Martin, Abingdon, OX14 4RY (GB); GRUDZIEN, Lukasz, Abingdon, OX14 4RY (GB)
(74) Representative: Lee, Nicholas John

(56) References cited:
- WO-A1-2011/001152
- WO-A1-2013/039889
- WO-A1-2018/204907

## Description

The present invention relates to formulations. In particular, it relates to pharmaceutical formulations of a bispecific protein comprising a soluble T cell receptor (TCR) and an scFv.

Advances in biotechnology have made it possible to make proteins for pharmaceutical applications. Because proteins are larger and more complex than traditional organic and inorganic drugs (e.g., possessing multiple functional groups in addition to complex three-dimensional structures), the formulation of such proteins poses special problems. For a protein to remain biologically active, a formulation must preserve intact the conformational integrity of at least a core sequence of the protein's amino acids while at the same time protecting the protein's multiple functional groups from degradation. Degradation pathways for proteins can involve chemical instability (e.g., any process which involves modification of the protein by bond formation or cleavage resulting in a new chemical entity) or physical instability (e.g., changes in the higher order structure of the protein). Chemical instability can result from deamidation, racemization, hydrolysis, oxidation, beta elimination or disulphide exchange. Physical instability can result from denaturation, aggregation, precipitation or adsorption, for example. The three most common protein degradation pathways are protein aggregation, deamidation and oxidation (Cleland et al Critical Reviews in Therapeutic Drug Carrier Systems 10(4): 307-377 (1993)).

Antibodies, including fragments and variants, are a class of therapeutic proteins for which successful pharmaceutical formulations have been described (Wang et al. J Pharm Sci. 2007 Jan;96(1):1-26.). However, the art relating to stable pharmaceutical formulations for bispecific proteins comprising a soluble T cell receptor (TCR) and an scFv is extremely limited. Because of the known complexities of protein formulation and the numerous differences between TCR bispecific proteins and antibodies, the technology developed for formulating antibodies cannot be expected to apply to these bispecific TCR proteins. For example, in antibody formulations, high protein concentration (i.e. above 1mg/ml) is desirable to minimise the volume of pharmaceutical product administered to a patient whilst maintaining the required therapeutic effect. TCR-scFv bispecific proteins are designed to be particularly potent at low protein concentration and therefore a formulation with a low protein concentration is desirable (i.e. below 1mg/ml). Low protein concentrations are particularly problematic since even a low level of aggregation or brief contact with vial surfaces (i.e. absorption loss) can result in a significant loss of protein activity.

Furthermore, unlike antibodies, TCRs are known to be inherently unstable in solution, meaning that maintaining protein stability at elevated temperatures (such as above -30°C or -20°C, e.g. 2-8°C) and/or during long-term storage is not straightforward. Stability of therapeutic proteins at elevated temperatures and during long-term storage are desirable features, which allow for convenient and low cost transport to and storage at clinical centres. Finally, glycosylation is an important consideration in antibody formulations since changes in glycosylation can affect the rate of protein degradation. TCR-scFv bispecific proteins are generally produced in *E.coli* and therefore not glycosylated. WO2018/204907 A1 discloses formulations of bispecific antibody constructs which are stable at elevated temperatures and which comprise surfactants and other excipients
There is a need to provide pharmaceutical formulations for TCR-scFv bispecific proteins. It is particularly desirable that such formulations maintain protein stability at low protein concentrations and during long-term storage at elevated temperatures.

In a first aspect, the present invention provides a pharmaceutical formulation comprising:
a therapeutically effective amount of a bispecific protein comprising a soluble T cell receptor (TCR) and an scFv; and
a buffer;
a surfactant;
a bulking agent; and
a stabiliser,
wherein the w/w ratio of surfactant to protein is in the range of 0.75:1 to 1.5:1 and the ratio of stabiliser to bulking agent is greater than 1:1, and wherein the surfactant is a polysorbate, the bulking agent is a polyol and the stabiliser is a disaccharide.

The inventors have found that a w/w ratio of surfactant to protein in the range of 0.75:1 to 1.5:1 provides a stable formulation. Surfactants are commonly used excipients in protein formulation to prevent both protein aggregation and absorption to surfaces. It has been shown in the art that a wide range of surfactant concentrations are suitable for preventing aggregation (between 0.02-2.0 mg/mL or 0.002-0.2%), and that higher concentrations are better at preventing precipitation (Wang, Eur J Pharm Biopharm. 2017 May;114: 263-277). Furthermore, it is common in known protein formulations that the weight of protein is in significant excess over the weight of surfactant; for example, in the formulation of marketed therapeutic antibodies trastuzumab (Heceptin^{®}) and bevacizumab (Avastin^{®}), the antibody is present in more than 50 fold excess over surfactant. The inventors have found that increasing or decreasing the w/w ratio of surfactant to protein outside of a narrow range of 0.75:1 to 1.5:1 in TCR-scFv bispecific protein formulations unexpectedly leads to substantial loss of protein stability. Without being bound by theory, the inventors hypothesise that the low concentration of protein in the formulation, as a result of the high potency of the drug, means that the protein is more sensitive to small changes in surfactant concentration.

The w/w ratio of surfactant to protein may be in the range of from 0.8:1 to 1.2:1, 0.9:1 to 1.1:1 or 0.95:1 to 1.05:1. The ratio may be 0.75:1, 0.8:1, 0.85:1, 0.9:1, 0.95:1, 1:1, 1.05:1, 1.1:1, 1.15:1, 1.2:1. A preferred ratio is 1:1.

The formulations of the present invention comprises a surfactant which is a polysorbate. Examples of pharmaceutically acceptable surfactants include polysorbates (e.g. polysorbates 20, 40, 60 or 80); polyoxamers (e.g. poloxamer 188); Triton; sodium octyl glycoside; lauryl-, myristyl-, linoleyl-, or stearyl-sulfobetaine; lauryl-, myristyl-, linoleyl- or stearyl-sarcosine; linoleyl-, myristyl-, or cetyl-betaine; lauroamidopropyl-, cocamidopropyl-, linoleamidopropyl-, myristamidopropyl-, palmidopropyl-, or isostearamidopropyl-betaine (e.g.lauroamidopropyl); myristamidopropyl-, palmidopropyl-, or iso stearamidopropyl-dimethylamine; sodium methyl cocoyl-, or disodium methyl oleyl-taurate, polyethyl glycol, polypropyl glycol, and copolymers of ethylene and propylene glycol. In the formulations of the present invention the surfactant is a polysorbate, such as polysorbate 20 (commercial name Tween^{®} 20) or polysorbate 80 (commercial name Tween^{®} 80). More preferably, the surfactant is polysorbate 20 (Tween^{®} 20). The surfactant may be present at a w/v concentration of 0.1% or less. For example, 0.9%, 0.8%, 0.7%, 0.6%, 0.5%, 0.4%, 0.3%, 0.2%. The concentration of surfactant may be 0.02%. The concentration of surfactant may be 0.05%. It is preferred of the surfactant is polysorbate 20, present at a concentration of 0.02%.

According to the invention, the ratio of stabiliser to bulking agent is greater than 1:1.

The inventors have found that the use of a bulking agent and stabiliser wherein the w/w ratio of stabiliser to bulking agent is greater than 1:1 provides a stable formulation that is lyophilisation ready. Mixtures comprising disaccharide stabilisers, such as trehalose or sucrose, and bulking agents, such as mannitol, are known to be suitable excipients for lyophilised protein formulations (Johnson, J Pharm Sci. 2002 Apr; 91(4):914-22.). Mannitol readily forms a crystalline structure and promotes the formation of a stable cake that does not collapse during drying. However, in the crystalline state, mannitol is not able to interact with protein, potentially leading to aggregation. The addition of sucrose, which forms an amorphous phase, can prevent aggregation. It has been proposed in the art that a weight ratio of at least 3:1 mannitol to sucrose is required to provide a sufficient balance between cake stability and prevention of aggregation; lower ratios have been shown to inhibit mannitol crystallisation (Jena et al, Pharm Res. 2016 Jun;33(6):1413-25; Johnson et al, J Pharm Sci. 2002 Apr;91(4):914-22). Contrary to the teachings in the art, the inventors have found that a ratio of bulking agent (i.e. mannitol) to stabiliser (i.e. sucrose/trehalose) of less than 1:1 (preferably 1:5) produces a stable cake that minimises aggregation. The inventors hypothesise that, when in excess, crystalline mannitol may cause excessive strain on the structure of the TCR-scFv bispecific protein in the lyophilised state, subsequently leading to unfolding, denaturation and aggregation.

The w/w ratio of stabiliser to bulking agent may preferably be in the range of from 3:1 to 7:1, more preferably 4:1 to 6:1. For example, the ratio may be 2:1 or 3:1 or 4:1 or 5:1 or 6:1. The ratio of stabiliser to bulking agent may preferably be about 5:1, for example from 5.5:0.9 to 4.5:1.1. The ratio of stabiliser to bulking agent may more preferably be 5:1. The concentration of stabiliser may be in the range of from 4.5. to 5.5 % (w/v), for example 5 %. The concentration of bulking agent used in the formulation may be between 0.9 and 1.1 % (w/v), for example 1 %.

Bulking agents are pharmaceutically acceptable compounds that function to add bulk to a lyo cake. Compounds that function as a bulking agent are known in the art and include polyols such as mannitol or sorbitol, carbohydrates, simple sugars, polymers, or proteins. According to the invention the bulking agent is a polyol. Preferably, the bulking agent is mannitol. Preferably, mannitol is present at a concentration of 1%.

Stabilisers are pharmaceutically acceptable compounds that function as a lyoprotectant/cytoprotectant upon freezing or lyophilisation (freeze-drying) and therefore prevent aggregation and/ or another chemical or physical instabilily. Compounds that function as stabilisers are known in the art. Example of stabilisers include sugars, sugaralcohols and amino acids. According to the invention, the stabiliser is a disaccharide. Preferably, the stabiliser is trehalose or sucrose. The stabiliser may be a mixture of trehalose and sucrose. Preferably, the stabiliser is trehalose present at a concentration of 5%. In a preferred formulation of the invention, the stabiliser is trehalose and the bulking agent is mannitol. The w/w ratio of trehalose to mannitol is preferably 5:1.

The formulation of the present invention further comprises a buffer. Pharmaceutically acceptable buffers are known in the art. Examples include phosphate, citrate, lysine, histidine, acetate, succinate and tris buffers. Any of these can be used in the present invention. A preferred buffer is phosphate-citrate. The concentration of buffer may be from about 20 mM to about 50 mM, for example 20 mM, 30 mM, 40 mM or 50 mM. Preferably, the buffer concentration is 50 mM. Preferably, the formulation comprises 50 mM phosphate-citrate buffer.

The optimal pH of a pharmaceutical formulation is dependent on the primary sequence of the protein and its concentration, as well as the identity and concentration of the other excipients. In a preferred formulation of the present invention, the pH is in the range of from about pH 6 to about pH 7, preferably 6.3-6.7. For example, the pH may be pH 6.0, or pH 6.1, or pH 6.2, or pH 6.3, or pH 6.4, or pH 6.5, or pH 6.6, or pH 6.7, or pH 6.8, or pH 6.9 or pH 7.0 pH 6.5 is preferred. pH 6.0 is also preferred.

The formulation may be an aqueous formulation. Preferably the aqueous carrier is one which is pharmaceutically acceptable (safe and non-toxic for administration to a human). The aqueous carrier may be distilled or sterile water. In some embodiments, the aqueous formulation may be frozen. Alternatively, the aqueous formulation may be lyophilised to produce a lyophilised formulation. Suitable protocols and methods for preparing lyophilized pharmaceutical formulations from liquid formulations are known in the art. Methods for reconstitution of lyophilised formulation prior to administration are known in the art. In brief, freeze-drying is accomplished by freezing the formulation and subsequently subliming ice from the frozen content at a temperature suitable for primary drying. Typically, primary drying will range from about -30 to 25° C (provided the product remains frozen during primary drying) at a suitable pressure, ranging typically from about 0.05 mBar to about 0.5 mBar. The formulation, size and type of the container holding the sample (e.g., glass vial) and the volume of liquid will mainly dictate the time required for drying, which can range from a few hours to several days. A secondary drying stage may be carried out at about 0-40° C, depending primarily on the type and size of container and the type of protein employed. The time and pressure required for secondary drying will be that which produces a suitable lyophilized cake, dependent, e.g., on the temperature and other parameters. The secondary drying time is dictated by the desired residual moisture level in the product and typically takes at least about 5 hours (e.g. 15-24 hours). The pressure may be the same as that employed during the primary drying step. Freeze-drying conditions can be varied depending on the formulation and vial size.

Pharmaceutical formulations of the invention are preferably stable. The term 'stability' or 'stable' as used herein means a formulation in which one or more biophysical and/or chemical characteristics of the bispecific protein is/are essentially unchanged when stored at 2-8°C for extended periods. By extended periods, it is meant up to at least 2 years, more preferably up to at least 5 years or longer. Additional or alternative conditions may be used as an indicator of the potential long-term stability of the formulation at 2-8°C. For example, stable may mean that the protein is essentially unchanged following a defined number of freeze thaw cycles, such as at least 5, or at least 10 freeze thaw cycles; or stable may mean that that the protein is essentially unchanged following shorter term storage at elevated temperatures, such as at a temperature of about 30°C for at least one month.

The stability of the formulation may be assessed by examining one or more biophysical or chemical characteristics including but not limited to, appearance (colour and turbidity), protein concentration, pH, presence of subvisible particles, aggregation, denaturation and protein activity. The skilled person is aware of various analytical techniques that may be used to assess stability of a protein formulation. Examples include but are not limited to, visual inspection, nephelometry, light obscuration, absorbance at 280 nm, capillary gel electrophoresis (CGE), anion exchange chromatography (AlEX), size exclusion chromatography (SEC), protein activity assays. For lyophilised samples, additional analytical techniques may include assessment of the physical stability of the cake and determination of moisture content (e.g. via Karl Fischer assessment). Specific techniques are further provided in the exemplification section. Preferably, stability of the formulation is determined by assessing one or more, and preferably all, of the following characteristics; pH, visual appearance, protein concentration, presence of subvisible particles, CGE, SEC, AIEX and protein activity.

The bispecific protein comprises a soluble TCR and a scFv antibody fragment, preferably fused via a linker. Linker sequences are usually flexible, in that they are made up of amino acids such as glycine, alanine and serine which do not have bulky side chains likely to restrict flexibility. Usable or optimum lengths of linker sequences are easily determined. Often the linker sequence will by less than about 12, such as less than 10, or from 5-10 amino acids in length. TCRs consist of two disulphide linked chains. Each chain (alpha and beta) is generally regarded as having two domains, namely a variable and a constant domain (termed TRAV/TRBV and TRAC/TRBC respectively). The variable domain of each chain is located N-terminally and comprises three Complementarity Determining Regions (CDRs) embedded in a framework sequence. The CDRs comprise the recognition site for peptide-MHC binding. Soluble TCRs may comprise TRAV and TRBV and may preferably be human. The soluble TCR may contain mutations relative to a natural or wild type (preferably human) TCR such that it has supra-physiological affinity for antigen (examples of antigens include NY-ESO, MAGEA4 or PRAME). The soluble TCR may be a heterodimeric αβ TCR polypeptide pair wherein the α and β polypeptides each have TCR variable and constant regions, but lack TCR transmembrane and cytoplasmic regions. A non-native disulfide bond between residues of the constant regions of the TCR α and β polypeptides may be present. The soluble TCR may recognise an antigen that is presented on diseased cells, such as cancer cells or virally or bacterially infected cells. The two chains of a soluble TCR may be produced as a single chain (WO2004/033685) or as a disulphide linked dimer (WO03/020763). In a specific embodiment, the soluble TCR comprises an alpha beta heterodimer having a TRAV and TRBV region and an extracellular alpha chain TRAC constant domain sequence and an extracellular beta chain TRBC1 or TRBC2 constant domain sequence. The TRAC or TRBV sequences may be modified by truncation or substitution to delete the native disulphide bond between Cys4 of exon 2 of TRAC and Cys2 of exon 2 of TRBC1 or TRBC2. The TRAC or TRBV sequence(s) may be modified by substitution of cysteine residues for Thr 48 of TRAC and Ser 57 of TRBC1 or TRBC2, the said cysteines forming a disulphide bond between the alpha and beta constant domains of the TCR. The constant regions of the α and β polypeptides may be linked by the native disulphide bond between Cys4 of exon 2 of TRAC*01 and Cys2 of exon 2 of TRBC1 or TRBC2. The soluble TCR may be a single chain αβ TCR polypeptide of the Vα-L-Vβ, Vβ-L-Vα, Vα-Cα-L-Vβ, or Vα-L-Vβ-Cβ type wherein Va and Vβ are TCR α and β variable regions respectively, Ca and Cβ α are TCR α and β constant regions respectively, and L is a linker sequence.

The term "single-chain Fv" or "scFv" refers to antibody fragments comprising the VH and VL domains of an antibody, wherein these domains are present in a single polypeptide chain. Generally, the Fv polypeptide further comprises a polypeptide linker between the VH and VL domains which enables the scFv to form the desired structure for antigen binding. The scFv may be derived from a human antibody or it may have been humanised. The scFv may be derived from an antibody that recognises CD3. In a specific embodiment, the anti-CD3 scFv may be derived from UCHT1 antibody (Shalaby et al. J Exp Med. 1992 Jan 1;175(1):217-25, US5821337). The scFv may be fused to the N- or C-terminus of the soluble TCR. It is preferred if the scFv is fused to the N terminus of the soluble TCR (WO2010/133828).

The TCR-scFv bispecific protein may be present at a concentration of less than 1 mg/ml, less than 0.5 mg/ml and preferably less than 0.3 mg/l. The concentration of the TCR-scFv bispecific protein may be in the range of from 0.05 to 0.5 mg/ml or 0.1 to 0.3 mg/ml or may be 0.5, 0.4, 0.3, 0.2 or 0.1 mg/ml. A preferred concentration is 0.2 mg/ml.

Specific TCR-scFv bispecific proteins that may be included in the formulations of the present invention include those disclosed in WO2011001152, WO2017/109496, WO2017/175006 and WO2018234319. A particular TCR-scFv bispecific protein of WO2011/001152 has: an alpha chain of SEQ ID No: 45, wherein amino acids 1-109 are replaced with SEQ ID No. 8, and the amino acid at position 1 is A and the C terminus of the alpha chain is truncated by 8 amino acids from F196 to S203 inclusive, based on the numbering of SEQ ID No: 45; and a beta chain of SEQ ID No: 36, in which residues 259-370 correspond to SEQ ID No. 27, amino acids at position 1 and 2 are A and I respectively. A formulation comprising such a TCR-scFv bispecific protein may preferably have a pH of 6.5.

A specific formulation in accordance with the invention comprises:
a TCR-scFv bispecific protein which has: an alpha chain of SEQ ID No: 45 of WO2011001152, wherein amino acids 1-109 are replaced with SEQ ID No. 8 of WO2011001152, and the amino acid at position 1 is A and the C terminus of the alpha chain is truncated by 8 amino acids from F196 to S203 inclusive, based on the numbering of SEQ ID No: 45; and a beta chain of SEQ ID No: 36 of WO2011001152, in which residues 259-370 correspond to SEQ ID No. 27 of WO2011001152, amino acids at position 1 and 2 are A and I respectively and is present at a concentration of 0.2 mg/ml;
50 mM phosphate-citrate buffer,
5% trehalose,
1% mannitol, and
0.02% w/v polysorbate 20
and has a pH of 6.5.

In an alternative preferred embodiment, the formulation comprises
0.5 mg/ml TCR-scFv bispecific protein, such as described in WO2017/109496,
50 mM phosphate-citrate buffer,
5% trehalose,
1% mannitol, and
0.05% w/v polysorbate 20
and has a pH of 6.0.

In a further alternative preferred embodiment, the formulation comprises
0.2 mg/ml TCR-scFv bispecific protein, such as described in WO2017/175006,
50 mM phosphate-citrate buffer,
5% trehalose,
1% mannitol, and
0.02% w/v polysorbate 20,
and has a pH of 6.0.

Preferably the bispecific protein in the formulations of the present invention is non-glycosylated.

In a further instance, the disclosure also provides a method of making a pharmaceutical formulation comprising:
formulating (i) a therapeutically effective amount of a bispecific protein comprising a soluble T cell receptor (TCR) and an scFV and (ii) a surfactant,
wherein the w/w ratio of surfactant to protein is in the range of 0.75:1 to 1.5:1.

In a further instance, the disclosure also provides a method of making a pharmaceutical formulation comprising:
formulating (i) a therapeutically effective amount of a bispecific protein comprising a soluble T cell receptor (TCR) and an scFV, (ii) a bulking agent and (iii) a stabiliser,
wherein the ratio of stabiliser to bulking agent is greater than 1:1.

The invention also provides a formulation of the invention for use in medicine. The formulation may be for use in a method for treating diseases including cancer or infectious diseases. The cancer may be melanoma (including but not limited to cutaneous melanoma or uveal melanoma (also known as ocular melanoma), Lentigo maligna melanoma, superficial spreading melanoma, ascral lentiginous melanoma, mucosal melanoma, nodular melanoma, polypoid melanoma, desmoplastic melanoma, amelanotic melanoma, soft-tissue melanoma, small cell melanoma with small nevus-like cells and spitzoid melanoma. Alternatively, the cancer may be of the breast (including triple negative), ovary, endometrium, oesophagus, lung (NSCLC and SCLC), bladder, colon, stomach, liver, pancreas, prostate, connective tissue (i.e. sarcoma) or the head and neck; or the cancer may be a leukemia or lymphoma.

In a preferred embodiment the formulation may be administered by injection, such as intravenous injection. The formulation may be administered via continuous infusion (such as over several hours, days, or weeks) or via intermittent infusion (such as once, twice or three times per week, or less frequently).

Alternatively, the formulation may be adapted for administration by any other appropriate route, including, intratumoral subcutaneous, intramuscular, intrathecal, enteral (including oral or rectal), inhalation or intranasal.

In a further aspect, the invention provides the formulation of the invention for use in a method of treating cancer, preferably melanoma. The cancer may be in a human subject.

Preferred features of any one aspect of the invention are for each other aspect of the invention *mutatis mutandis.*

The invention will now be described in the following non-limiting examples. Reference is made to the accompanying drawings in which:
**Figure 1** provides results of SE-UPLC, AE-HPLC and CGE analyses for a formulation comprising 0.2 mg/ml of a TCR-scFv bispecific protein, 50 mM phosphate-citrate pH 6.5, 5% trehalose, 1% mannitol, and 0.02% Tween 20.
**Figure 2** provides results of SE-UPLC, AE-HPLC and CGE analyses for a formulation comprising 0.2 mg/ml of an alternative TCR-scFv bispecific protein, 50 mM phosphate-citrate pH 6.5, 5% trehalose, 1% mannitol, and 0.02% Tween 20.
**Figure 3** provides result of light obscuration analyses for a formulation comprising 0.2 mg/ml of a TCR-scFv bispecific protein, 50 mM phosphate-citrate pH 6.5, 5% trehalose, 1% mannitol, and either 0.06%, 0.04%, 0.02%, 0.01%, or 0.005% Tween 20.
**Figure 4** provides result of a Design of Experiments (DoE) analysis for a formulation comprising 0.2 mg/ml of a TCR-scFv bispecific protein, 50 mM phosphate-citrate pH 6.5, 5% trehalose, 1% mannitol, and either 0.02%, 0.03%, 0.04%, 0.05%, 0.06%, 0.07%, or 0.08% Tween 20.
**Figure 5** **p**rovides results of SE-UPLC, AE-HPLC and CGE analyses for a formulation comprising 0.5 mg/ml of a TCR-scFv bispecific protein, 50 mM phosphate-citrate pH 7.5, 120mM NaCl and 0.05% Tween 20.
**Figure 6** provides results of turbidity analysis for a formulation comprising 0.2 mg/ml of a TCR-scFv bispecific protein, 50 mM phosphate-citrate pH 6.5, 5% mannitol 0.02% Tween in the presence or absence of 1% sucrose.

### Examples

### Example 1 - stable aqueous formulation for a soluble TCR-scFv bispecific protein

### A)

A bispecific protein comprising a soluble TCR fused to an anti-CD3 scFv (an alpha chain of SEQ ID No: 45 of WO2011/001152, wherein amino acids 1-109 are replaced with SEQ ID No. 8 of WO2011001152, and the amino acid at position 1 is A and the C terminus of the alpha chain is truncated ; by 8 amino acids from F196 to S203 inclusive, based on the numbering of SEQ ID No: 45 and a beta chain of SEQ ID No: 36 of WO2011/001152, in which residues 259-370 correspond to SEQ ID No. 27 of WO2011/001152, amino acids at position 1 and 2 are A and I respectively) was prepared using known methods and buffer-exchanged into formulation buffer using size exclusion chromatography (SEC). The protein was subsequently diluted to a final concentration of 0.20 ± 0.02 mg/mL using formulation buffer and filled into bottles in a laminate airflow (LAF) bench.

The formulation buffer comprised 50 mM phosphate-citrate, 5% trehalose, 1% mannitol, and 0.02% Tween 20. The pH was 6.5.

Protein stability was monitored over 24 months at either <-60°C or +5°C. Stability at each temperature was assessed using various analytical methods including capillary gel electrophoresis (CGE), anion exchange high performance liquid chromatography (AE-HPLC) and size exclusion ultra performance liquid chromatography (SE-UPLC). Further assessments included visual inspection, measurement of protein content and pH, detection of sub-visible particles by light obscuration and activity assays to confirm function. Protein stability was also demonstrated over 10 freeze thaw cycles.

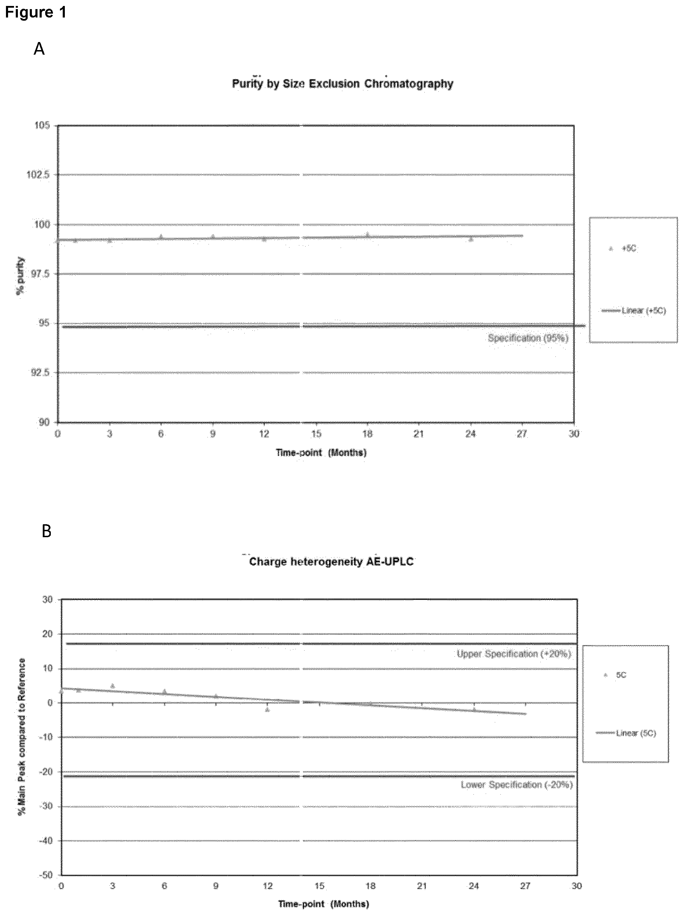

SE-UPLC was used to detect the presence of multimers and other higher molecular weight species and was carried out on an UPLC system using an 1.7 µm, 4.6x300mm UPLC SE column, with a 0.2M phosphate pH 7.5 mobile phase flowing at 0.2 ml/min isocratic. The method comprises the separation of protein components in the test sample on a SE column following injection of a suitable amount onto the column using detection by fluorescence on an UPLC system. Figure 1 A shows an assessment of purity measured by SE-UPLC at eight time points over the course of 24 months at +5°C. The purity at 0 months was 99.2% and at 24 months 99.3%. Minimum acceptance criteria = >95%

AE-HPLC was used to detect charge-based degradations and carried out on an HPLC system using a 2x250mm weak AE column, with a phosphate pH 8.2 mobile phase flowing at 0.2 ml/min. The protein was eluted with a NaCl gradient in phosphate pH 8.2 buffer. The method comprises the separation of protein components in the test sample on an AE column following injection of a suitable amount onto the column using detection by fluorescence on an HPLC system. Figure 1B shows an assessment of charge status as measured by AE-HPLC at eight time points over the course of 24 months at +5°C. The % main peak at 0 months was +3.4% compared to reference, at 24 months the reading was - 1.9%, demonstrating the product was stable in this formulation (acceptance criteria within ±15% of reference).

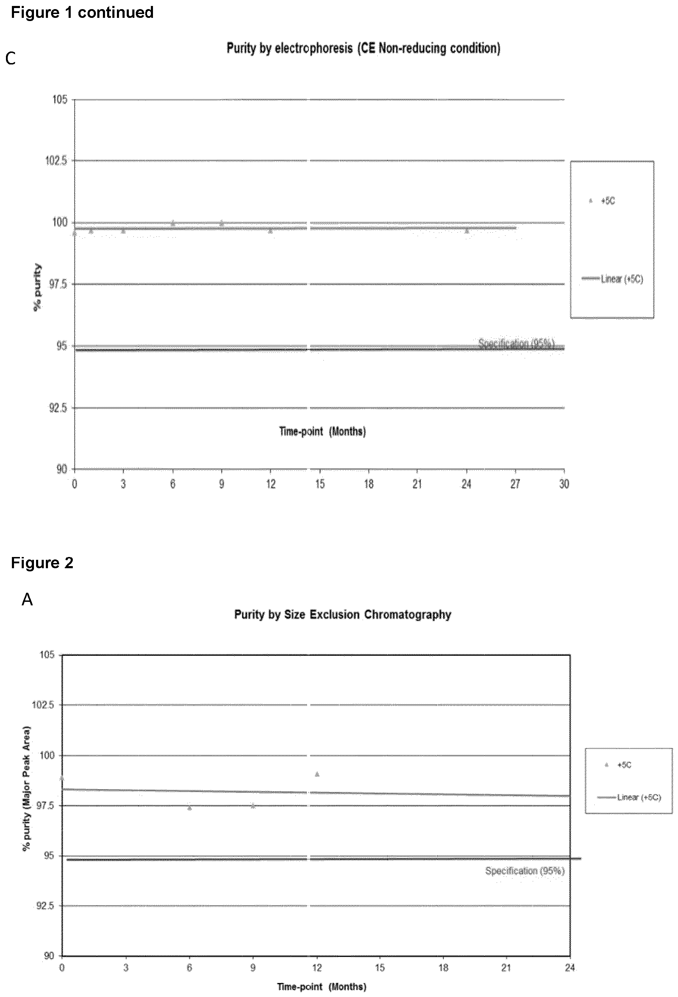

CGE was used to determine the presence of low molecular weight impurities and degradants such as fragments. The method was carried out using both chip and standard CGE. In the chip method samples were prepared to a final concentration of 0.2 mg/mL and heated for 5 minutes at 70°C. After cooling the denatured samples are loaded onto the chip and separated on a Bioanalyzer (Agilent technologies). Standard CGE was run on PA 800 plus instrument with a Diode array detector (Beckman Coulter) using reagents from the Proteolab SDS MW analysis kit, and a separation capillary (57 cm x 50 µm ID, bare fused-silica). Samples were prepared at 0.2 mg/mL before diluting in SDS Sample buffer and heating for 5 minutes at 70 °C. Analysis is read at 220nm and the migration time and % corrected peak area is recorded. Minimum acceptance criteria = >95%
Figure 1C shows an assessment of purity by CGE at seven time points over the course of 24 months at +5°C. The purity at 0 months was 99.6%, at T=24 months 99.7%, demonstrating the product was stable in this formulation.

Assessment of stability following multiple freeze/thaw cycles was used as a further indicator of long-term stability. Freeze thaw cycles were performed by rapidly freezing the sample to below <-60 and then incubating at room temperature (approx. 22°C). Stability was assessed by SE-UPLC as described above. The results showed a purity of 99.6%, before exposure to 10 freeze thaw cycles and a purity of 99.5% afterwards, indicating that the product was stable in this formulation.

Visual analysis was carried out following principles of pharmacopoeial method (Ph. Eur.2.2.1, 2.2.2 and 2.9.20). A clear colorless solution, essentially free from particles, was observed at 0 months and after 24 months at +5°C. In addition, no visual changes were detected following 10 freeze thaw cycles.

Protein concentration and pH were within +/- 0.02 mg/ml and 0.2 pH units of the stated values after 24 months.

Assessment of subvisible particles by laser light obscuration showed that particles of ≥25µm were present at ≤600/vial, and particles of ≥10µm were present at ≤ 6000/vial after 24 months (i.e. within acceptance criteria). Turbidity measurements were within an acceptable range (<3 NTU (standard of opalescence I).

These data demonstrate that the formulation stable during long term storage at temperatures between 2-8°C
A lyophilised formulation was prepared by freezing and then holding 0.6 ml samples in 2R glass vials at -45 °C for 2 h, followed by primary drying at -15 °C/0.07 mbar for 22 h then secondary drying at +40 °C/0.07mbar for 23 h. Samples were then sealed in the vials with rubber stoppers under vacuum. Lyophilised samples were stored long term at +5 °C. Samples were reconstituted by addition of 0.6ml sterile water for injection to the vial and mixing.

### B)

A second bispecific protein comprising an alternative soluble TCR fused to an anti-CD3 scFv (as set out in WO2017/175006) was prepared at a final concentration of 0.20 ± 0.02 mg/mL. The formulation buffer comprised 20 mM phosphate-citrate, 5% trehalose, 1% mannitol, and 0.02% Tween 20. The pH was 6.0.

Stability assessment was carried out using the same methods as described in part A, over a period of 12 months.

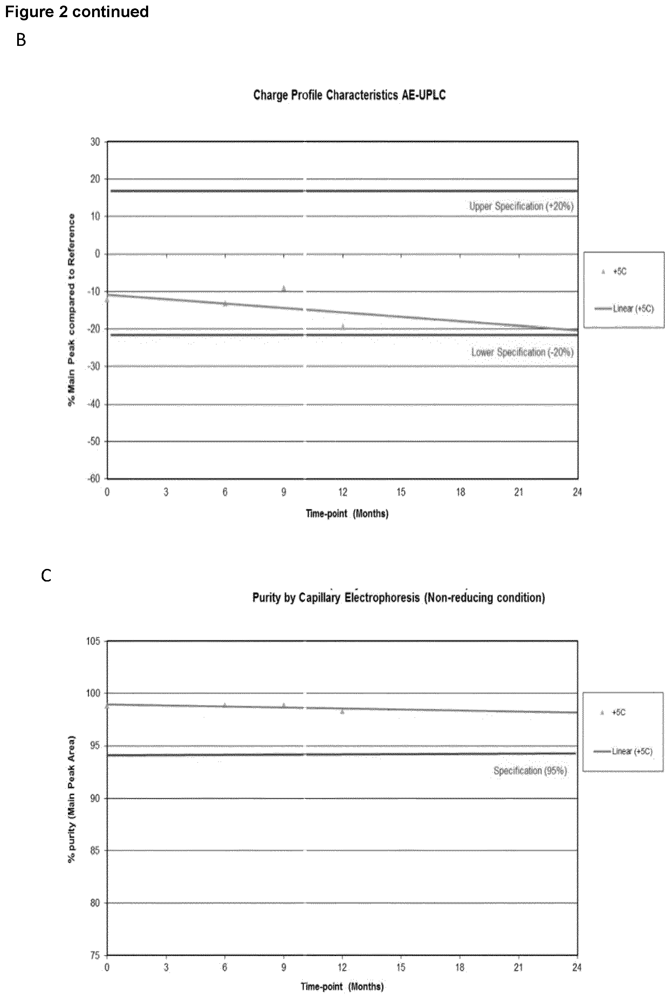

The SE-UPLC, AE-HPLC and CGE data are shown in Figures 2A-C respectively. Purity by SE-UPLC was 98.9% at 0 months and 99.3% at 12 months. The charge status by AE-HPLC was -12.2 % main peak compared to the reference at 0 months and -19.5% at 12 months (acceptance criteria within ±20% of reference). Purity by CGE was 98.8% at 0 months and 98.3% at 12 months. In each case, extrapolation of data indicates stability can be expected ≥24 months.

Stability was also demonstrated by SE-UPLC following 10 freeze thaw cycles.

These data demonstrate that the formulation is stable during long term storage at temperatures between 2-8°C.

### Example 2 - Formulation optimisation

### A - Detergent Protein Ratio

The weight ratio between detergent and protein was found to be a critical factor in producing a stable formulation.

Evidence for this was obtained from laser light obscuration (LO) measurements which assessed the presence of subvisible particles of size 2 µm and greater, following 5 freeze thaw cycles. The formulation buffer comprised the TCR-scFv bispecific protein of Example 1A, 50 mM phosphate-citrate, 5% trehalose, 1% mannitol, and Tween 20 at one of 5 different concentrations. The pH was 6.5. The protein concentration was kept constant at 0.2 mg/ml. The assay was carried out on a PAMAS SVSS-C or equivalent particle sizing instrument. Samples were analysed neat, with a pre-run volume of 0.4 ml and measurement volume of 0.1 ml carried out in triplicate. Particle counts for particles ≥2 µm, ≥10 µm and ≥25 µm were determined.

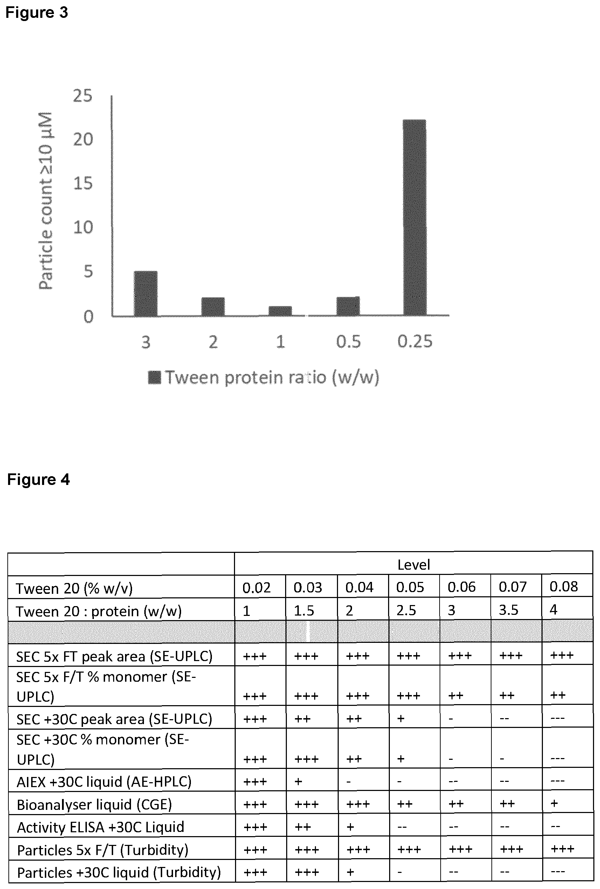

Figure 3 shows that the lowest counts of particles of size ≥10 µm, and therefore the highest stability, was obtained when the ratio of Tween-20 to protein was 1.

Further evidence of the importance of the detergent protein ratio was obtained from a Design of Experiments (DoE) study. In this experiment, stability of the bispecific protein of Example 1A was examined in 32 different conditions at a fixed protein concentration of 0.2 mg/ml. Variables included the detergent concentration. In each case the formulations were stored for 1 month at +30°C ±3°C and stability monitored at the elevated temperature and through 5x freeze thaw cycles. The effect of Tween 20 concentration (and therefore Tween 20 /protein w/w ratios between 1 and 4) was assessed across a number of analytical methods including SE-UPLC, AE- HPLC and CGE. In addition, bispecific protein activity was assessed using ELISA. The results are summarised in Figure 4.

### B - Mannitol + Sucrose / Trehalose

The inclusion of both mannitol and either sucrose or trehalose was found to be a critical factor in the formulation, and necessary to provide a formulation that is lyophilisation ready.

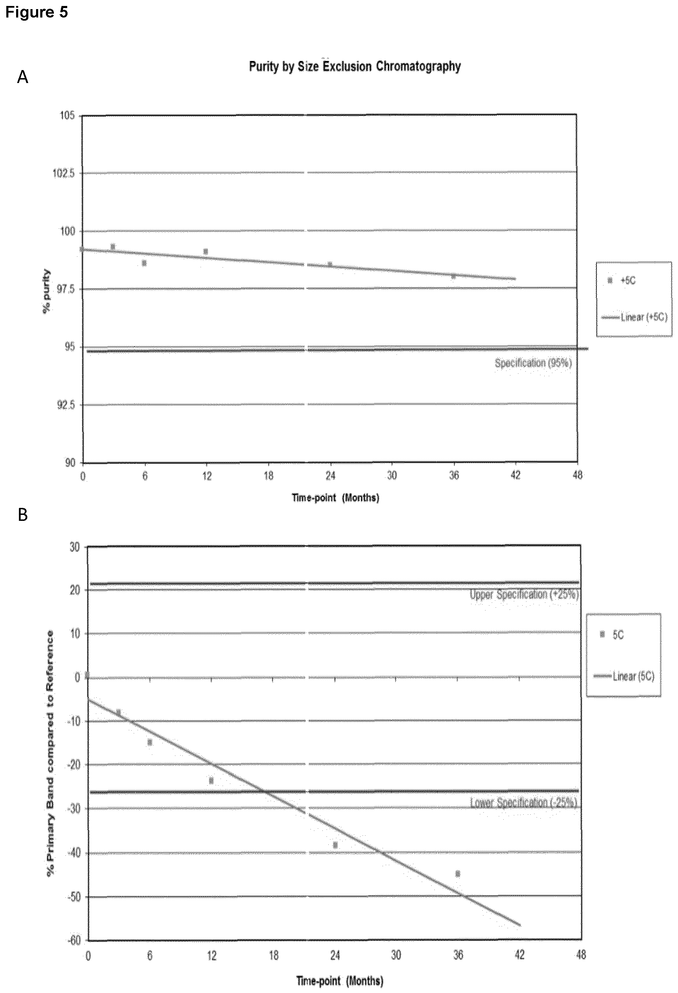

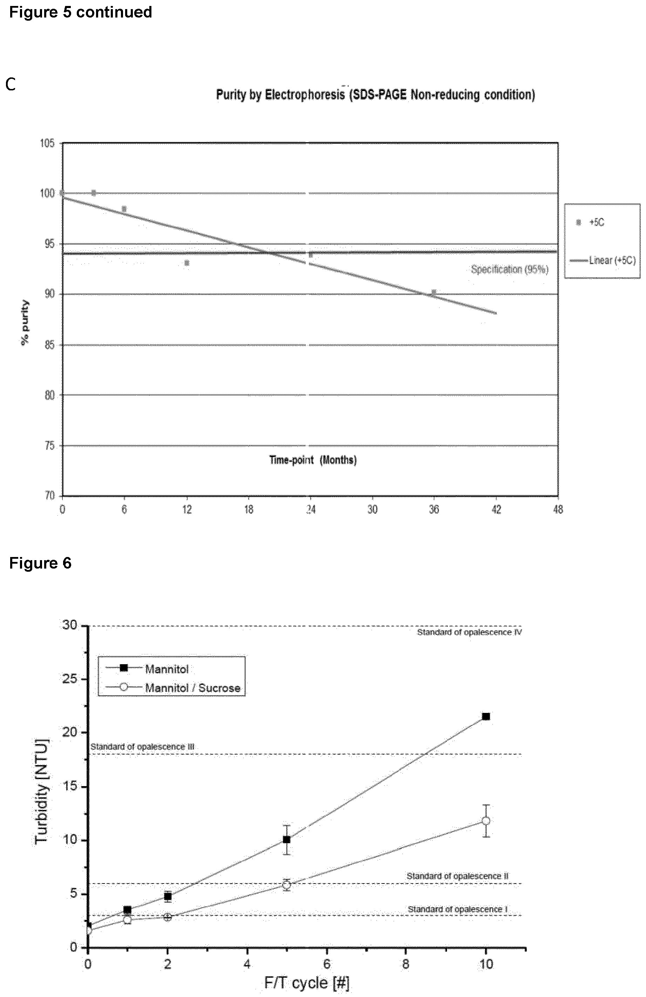

The bispecific protein of Example 1A was prepared in formulation buffer comprising 50 mM Phosphate-citrate, 120 mM NaCl and 0.05% Tween 20, at a final protein concentration of 0.5 mg/ml. The pH was 7.5. Stability was assessed using the same methods as described in Example 1. Figure 5A-C shows SE-UPLC, AE-HPLC and CGE data over the course of 36 months at +5°C. The data indicate that the formulation was not stable after 12 months. Using DoE methodology it was subsequently found that the addition of either 5% mannitol or 5% sucrose improved stability. The omission of NaCl was also preferable. However, a formulation comprising 5% mannitol resulted in high levels of aggregation following 10 freeze thaw cycles as determined by laser light obscuration (LO) and turbidity (NTU) measurements. Turbidity was determined by nephelometry using a turbidimeter and calibration against standards of known turbidity.

Figure 6 demonstrates that the addition of 1% sucrose reduced freeze thaw induced aggregation.

To further investigate lyophilisation readiness, a formulation was prepared comprising 50 mM phosphate-citrate, 5% mannitol, 1 % sucrose, 0.02% Tween 20, at a final protein concentration of 0.2 mg/ml. The pH was 6.5. Samples were lyophilised in glass DP lyo vials and stored at 50°C 25°C and 40°C for 1, 2 and 3 months. The samples were subsequently rehydrated by adding 0.5ml ultra pure water and incubated for 1h at room temperature before being assessed using a number of techniques including turbidity, laser light obscuration, SE-UPLC, AE-HLPC, CGE and protein activity. Increased aggregation and significant loss of protein activity was observed at higher temperatures, indicating that the formulation was not suitable for long-term storage.

To improved long term stability and maintain liquid stability, it was subsequently found that the ratio of mannitol to either sucrose was important for maintaining stability and that trehalose could be interchanged with sucrose. Formulations of the bispecific protein of Example 1A comprising two different mannitol/sucrose ratios were assessed following lyophilisation and storage at 25°C or 40°C for four weeks. Assessment of stability was carried out using a number of techniques including turbidity, laser light obscuration, SE-UPLC, AE-HLPC, and protein activity. Turbidity data are shown in the table below and indicate that higher concentrations of mannitol relative to sucrose result in increased aggregation.

| Formulation | Turbidity (NTU) 4 weeks | Turbidity (NTU) 4 weeks |
|---|---|---|
| *50 mM phosphate-citrate, 0.02% Tween 20, protein 0.2 mg*/*ml, pH 6.5, plus:* | at +25°C | at +40°C |
| 5% mannitol/1% sucrose | 18 | 30 |
| 1% mannitol/5% sucrose | 0 | 0 |
| 1% mannitol/5% trehalose | 0 | 0 |

## Claims

1. A pharmaceutical formulation comprising:
a therapeutically effective amount of a bispecific protein comprising a soluble T cell receptor (TCR) and an scFV;
a buffer;
a surfactant;
a bulking agent; and
a stabiliser,
wherein the w/w ratio of surfactant to protein is in the range of 0.75:1 to 1.5:1 and the ratio of stabiliser to bulking agent is greater than 1:1, and
wherein the surfactant is a polysorbate, the bulking agent is a polyol and the stabiliser is a disaccharide.

2. The formulation of claim 1, wherein
the w/w ratio of surfactant to protein is 1:1; and/or
the ratio of stabiliser to bulking agent is greater than 1.5:1, optionally wherein the ratio of stabiliser to bulking agent is in the range of from 3:1 to 7:1.

3. The formulation of claim 1 or claim 2, wherein the surfactant is present at a w/v concentration of 0.1% or less, optionally wherein the surfactant is present at a w/v concentration of 0.02%.

4. The formulation of any preceding claim, wherein the polysorbate is polysorbate 20.

5. The formulation of any preceding claim, wherein the w/v concentration of bulking agent is between 0.9% and 1.1%, optionally wherein the w/v concentration of bulking agent is 1%.

6. The formulation of any preceding claim, wherein the polyol is mannitol.

7. The formulation of any preceding claim, wherein
the w/v concentration of stabiliser is in the range of 4.5% to 5.5%, optionally wherein the w/v concentration of stabiliser is 5%, and/or
the stabiliser is sucrose or trehalose, or a mixture of trehalose and sucrose.

8. The formulation of any preceding claim, wherein
the concentration of buffer is from 20 mM to 50 mM, optionally wherein the concentration of buffer is 50 mM, and/or
the buffer is phosphate/citrate, and/or
the formulation has a pH in the range of from 6 to 7, optionally wherein the pH is 6.0, 6.5 or 6.8.

9. The formulation of any preceding claim, wherein the concentration of the TCR-scFv bispecific protein is in the range of 0.05 to 0.5 mg/mL, optionally wherein the concentration of the TCR-scFv bispecific protein is 0.2 mg/mL.

10. The formulation of any preceding claim, wherein
the soluble TCR recognises an antigen that is presented on diseased cells, optionally wherein the antigen is presented on cancer cells, virally infected cells or bacterially infected cells, and/or
the soluble TCR contains mutations relative to a native or wildtype TCR such that it has supra-physiological affinity for an antigen, optionally wherein the antigen is PRAME.

11. The formulation of any preceding claim, wherein
(a) the TCR-scFv bispecific protein has:
(i) an alpha chain having the following sequence: 
GDAKTTQPNSMESNEEEPVHLPCNHSTISGTDYIHWYRQLPSQGPEYVIHGLTSNVNN
RMASLAIAEDRKSSTLILHRATLRDAAVYYCILILGHSRAGNYIATFGKGTKLSVIPNIQNP
DPAVYQLRDSKSSDKSVCLFTDFDSQTNVSQSKDSDVYITDKCVLDMRSMDFKSNSAV
AWSNKSDFACANAFNNSIIPEDT;
 and
(ii) a beta chain having the following sequence: 
AIQMTQSPSSLSASVGDRVTITCRASQDIRNYLNWYQQKPGKAPKLLIYYTSRLESGVP
SRFSGSGSGTDYTLTISSLQPEDFATYYCQQGNTLPWTFGQGTKVEIKGGGGSGGGG
SGGGGSGGGGSGGGSEVQLVESGGGLVQPGGSLRLSCAASGYSFTGYTMNWVRQA
PGKGLEWVALINPYKGVSTYNQKFKDRFTISVDKSKNTAYLQMNSLRAEDTAVYYCAR
SGYYGDSDWYFDVWGQGTLVTVSSGGGGSDGGITQSPKYLFRKEGQNVTLSCEQNL
NHDAMYWYRQDPGQGLRLIYYSQIMGDEQKGDIAEGYSVSREKKESFPLTVTSAQKNP
TAFYLCASSWWTGGASPISFGPGTRLTVTEDLKNVFPPEVAVFEPSEAEISHTQKATLV
CLATGFYPDHVELSWWVNGKEVHSGVCTDPQPLKEQPALNDSRYALSSRLRVSATFW
QDPRNHFRCQVQFYGLSENDEWTQDRAKPVTQIVSAEAWGRAD;
or
(b) the TCR-scFv bispecific protein has:
(i) an alpha chain having the following sequence: 
GDAKTTQPNSMESNEEEPVHLPCNHSTISGTDYIHWYRQLPSQGPEYVIHGLTSNVNN
RMASLAIAEDRKSSTLILHRATLRDAAVYYCILILGHSRLGNYIATFGKGTKLSVIPNIQNP
DPAVYQLRDSKSSDKSVCLFTDFDSQTNVSQSKDSDVYITDKCVLDMRSMDFKSNSAV
AWSNKSDFACANAFNNSIIPEDT;
 and
(ii) a beta chain having the following sequence: 
AIQMTQSPSSLSASVGDRVTITCRASQDIRNYLNWYQQKPGKAPKLLIYYTSRLESGVP
SRFSGSGSGTDYTLTISSLQPEDFATYYCQQGNTLPWTFGQGTKVEIKGGGGSGGGG
SGGGGSGGGGSGGGSEVQLVESGGGLVQPGGSLRLSCAASGYSFTGYTMNWVRQA
PGKGLEWVALINPYKGVSTYNQKFKDRFTISVDKSKNTAYLQMNSLRAEDTAVYYCAR
SGYYGDSDWYFDVWGQGTLVTVSSGGGGSDGGITQSPKYLFRKEGQNVTLSCEQNL
NHDAMYWYRQDPGQGLRLIYYSQIMGDEQKGDIAEGYSVSREKKESFPLTVTSAQKNP
TAFYLCASSWWTGGASPIRFGPGTRLTVTEDLKNVFPPEVAVFEPSEAEISHTQKATLV
CLATGFYPDHVELSWWVNGKEVHSGVCTDPQPLKEQPALNDSRYALSSRLRVSATFW
QDPRNHFRCQVQFYGLSENDEWTQDRAKPVTQIVSAEAWGRAD;
or
(c) the TCR-scFv bispecific protein has:
(i) an alpha chain having the following sequence: 
GDAKTTQPNSMESNEEEPVHLPCNHSTISGTDYIHWYRQLPSQGPEYVIHGLTSNVNN
RMASLAIAEDRKSSTLILHRATLRDAAVYYCILILGHSRLGNYIATFGKGTKLSVIPNIQNP
DPAVYQLRDSKSSDKSVCLFTDFDSQTNVSQSKDSDVYITDKCVLDMRSMDFKSNSAV
AWSNKSDFACANAFNNSIIPEDT;
 and
(ii) a beta chain having the following sequence: 
AIQMTQSPSSLSASVGDRVTITCRASQDIRNYLNWYQQKPGKAPKLLIYYTSRLESGVP
SRFSGSGSGTDYTLTISSLQPEDFATYYCQQGNTLPWTFGQGTKVEIKGGGGSGGGG
SGGGGSGGGGSGGGSEVQLVESGGGLVQPGGSLRLSCAASGYSFTGYTMNWVRQA
PGKGLEWVALINPYKGVSTYNQKFKDRFTISVDKSKNTAYLQMNSLRAEDTAVYYCAR
SGYYGDSDWYFDVWGQGTLVTVSSGGGGSDGGITQSPKYLFRKEGQNVTLSCEQNL
NHDAMYWYRQDPGQGLRLIYYSQIMGDEQKGDIAEGYSVSREKKESFPLTVTSAQKNP
TAFYLCASSWWTGGSAPIRFGPGTRLTVTEDLKNVFPPEVAVFEPSEAEISHTQKATLV
CLATGFYPDHVELSWWVNGKEVHSGVCTDPQPLKEQPALNDSRYALSSRLRVSATFW
QDPRNHFRCQVQFYGLSENDEWTQDRAKPVTQIVSAEAWGRAD,
preferably wherein the TCR-scFv bispecific protein has the alpha chain and beta chain sequences defined in part (b).

12. The formulation of any one of claims 1 to 10, wherein the bispecific protein has an alpha chain having the following sequence: 
AQQGEEDPQALSIQEGENATMNCSYKTSINNLQWYRQNSGRGLVHLILIRSNEREKHS
GRLRVTLDTSKKSSSLLITASRAADTASYFCATDGSTPMQFGKGTRLSVIANIQKPDPAV
YQLRDSKSSDKSVCLFTDFDSQTNVSQSKDSDVYITDKCVLDMRSMDFKSNSAVAWS
NKSDFACANAFNNSIIPEDT
 and a beta chain having the following sequence:

AIQMTQSPSSLSASVGDRVTITCRASQDIRNYLNWYQQKPGKAPKLLIYYTSRLESGVP
SRFSGSGSGTDYTLTISSLQPEDFATYYCQQGNTLPWTFGQGTKVEIKGGGGSGGGG
SGGGGSGGGGSGGGSEVQLVESGGGLVQPGGSLRLSCAASGYSFTGYTMNWVRQA
PGKGLEWVALINPYKGVSTYNQKFKDRFTISVDKSKNTAYLQMNSLRAEDTAVYYCAR
SGYYGDSDWYFDVWGQGTLVTVSSGGGGSDGGITQSPKYLFRKEGQNVTLSCEQNL
NHDAMYWYRQDPGQGLRLIYYSWAQGDFQKGDIAEGYSVSREKKESFPLTVTSAQKN
PTAFYLCASSWGAPYEQYFGPGTRLTVTEDLKNVFPPEVAVFEPSEAEISHTQKATLVC
LATGFYPDHVELSWWVNGKEVHSGVCTDPQPLKEQPALNDSRYALSSRLRVSATFWQ
DPRNHFRCQVQFYGLSENDEWTQDRAKPVTQIVSAEAWGRAD

13. The formulation of any preceding claim, comprising
50 mM phosphate-citrate buffer,
5% trehalose,
1% mannitol, and
0.02% w/v polysorbate 20,
wherein the formulation has a pH of 6.0, 6.5 or 6.8 and the bispecific protein is present at a concentration of 0.2 mg/ml.

14. The formulation of any of claims 1 to 12, comprising
20 mM phosphate-citrate buffer,
5% trehalose,
1% mannitol, and
0.02% w/v polysorbate 20,
wherein the formulation has a pH of 6.0, 6.5 or 6.8 and the bispecific protein is present at a concentration of 0.2 mg/ml.

15. The formulation of any preceding claim, wherein the formulation is aqueous or lyophilised.

16. The formulation of any preceding claim for use in medicine.

17. The formulation for use of claim 16, for use in a method for treating cancer.

18. The formulation for use of claim 17, wherein the cancer is melanoma, preferably uveal melanoma.

## Patentansprüche

1. Pharmazeutische Formulierung, umfassend:
eine therapeutisch wirksame Menge eines bispezifischen Proteins, das einen löslichen T-Zell-Rezeptor (TCR) und ein scFV umfasst;
einen Puffer;
ein Tensid;
einen Füllstoff; und
einen Stabilisator,
wobei das Gewichtsverhältnis von Tensid zu Protein im Bereich von 0,75:1 bis 1,5:1 liegt und das Verhältnis von Stabilisator zu Füllstoff größer 1:1 ist und
wobei es sich bei dem Tensid um ein Polysorbat handelt, es sich bei dem Füllstoff um ein Polyol handelt und es sich bei dem Stabilisator um ein Disaccharid handelt.

2. Formulierung nach Anspruch 1, wobei
das Gewichtsverhältnis von Tensid zu Protein 1:1 beträgt und/oder
das Verhältnis von Stabilisator zu Füllstoff größer 1,5:1 ist, gegebenenfalls wobei das Verhältnis von Stabilisator zu Füllstoff im Bereich von 3:1 bis 7:1 liegt.

3. Formulierung nach Anspruch 1 oder Anspruch 2, wobei das Tensid in einer w/v-Konzentration von 0,1 % oder weniger vorliegt, gegebenenfalls wobei das Tensid in einer w/v-Konzentration von 0,02 % vorliegt.

4. Formulierung nach einem vorhergehenden Anspruch, wobei es sich bei dem Polysorbat um Polysorbat 20 handelt.

5. Formulierung nach einem vorhergehenden Anspruch, wobei die w/v-Konzentration des Füllstoffs zwischen 0,9 % und 1,1 % liegt, gegebenenfalls wobei die w/v-Konzentration des Füllstoffs 1 % beträgt.

6. Formulierung nach einem vorhergehenden Anspruch, wobei es sich bei dem Polyol um Mannit handelt.

7. Formulierung nach einem vorhergehenden Anspruch, wobei
die w/v-Konzentration des Stabilisators im Bereich von 4,5 % bis 5,5 % liegt, gegebenenfalls wobei die w/v-Konzentration des Stabilisators 5 % beträgt und/oder
es sich bei dem Stabilisator um Saccharose oder Trehalose oder eine Mischung aus Trehalose und Saccharose handelt.

8. Formulierung nach einem vorhergehenden Anspruch, wobei
die Konzentration des Puffers 20 mM bis 50 mM beträgt, gegebenenfalls wobei die Konzentration des Puffers 50 mM beträgt und/oder
es sich bei dem Puffer um Phosphat/Citrat handelt und/oder
die Formulierung einen pH-Wert im Bereich von 6 bis 7 aufweist, gegebenenfalls wobei der pH-Wert 6,0, 6,5 oder 6,8 beträgt.

9. Formulierung nach einem vorhergehenden Anspruch, wobei die Konzentration des bispezifischen TCR-scFv-Proteins im Bereich von 0,05 bis 0,5 mg/ml liegt, gegebenenfalls wobei die Konzentration des bispezifischen TCR-scFv-Proteins 0,2 mg/ml beträgt.

10. Formulierung nach einem vorhergehenden Anspruch, wobei
der lösliche TCR ein Antigen erkennt, das auf erkrankten Zellen präsentiert wird, gegebenenfalls wobei das Antigen auf Krebszellen, virusinfizierten Zellen oder bakteriell infizierten Zellen präsentiert wird und/oder
der lösliche TCR Mutationen im Vergleich zu einem nativen oder Wildtyp-TCR enthält, sodass er eine supraphysiologische Affinität für ein Antigen aufweist, gegebenenfalls wobei es sich bei dem Antigen um PRAME handelt.

11. Formulierung nach einem vorhergehenden Anspruch, wobei
(a) das bispezifische TCR-scFv-Protein Folgendes aufweist:
(i) eine Alpha-Kette mit der folgenden Sequenz: 
GDAKTTQPNSMESNEEEPVHLPCNHSTISGTDYIHWYRQLPSQGPEYVIHGLTSN
VNNRMASLAIAEDRKSSTLILHRATLRDAAVYYCILILGHSRAGNYIATFGKGTK
LSVIPNIQNPDPAVYQLRDSKSSDKSVCLFTDFDSQTNVSQSKDSDVYITDKCVL
DMRSMDFKSNSAVAWSNKSDFACANAFNNSIIPEDT;
 und
(ii) eine Beta-Kette mit der folgenden Sequenz: 
AIQMTQSPSSLSASVGDRVTITCRASQDIRNYLNWYQQKPGKAPKLLIYYTSRLE
SGVPSRFSGSGSGTDYTLTISSLQPEDFATYYCQQGNTLPWTFGQGTKVEIKGGG
GSGGGGSGGGGSGGGGSGGGSEVQLVESGGGLVQPGGSLRLSCAASGYSFTGYTM
NWVRQAPGKGLEWVALINPYKGVSTYNQKFKDRFTISVDKSKNTAYLQMNSLRAE
DTAVYYCARSGYYGDSDWYFDVWGQGTLVTVSSGGGGSDGGITQSPKYLFRKEGQ
NVTLSCEQNLNHDAMYWYRQDPGQGLRLIYYSQIMGDEQKGDIAEGYSVSREKKE
SFPLTVTSAQKNPTAFYLCASSWWTGGASPISFGPGTRLTVTEDLKNVFPPEVAV
FEPSEAEISHTQKATLVCLATGFYPDHVELSWWVNGKEVHSGVCTDPQPLKEQPA
LNDSRYALSSRLRVSATFWQDPRNHFRCQVQFYGLSENDEWTQDRAKPVTQIVSA
EAWGRAD;
oder
(b) das bispezifische TCR-scFv-Protein Folgendes aufweist:
(i) eine Alpha-Kette mit der folgenden Sequenz: 
GDAKTTQPNSMESNEEEPVHLPCNHSTISGTDYIHWYRQLPSQGPEYVIHGLTSN
VNNRMASLAIAEDRKSSTLILHRATLRDAAVYYCILILGHSRLGNYIATFGKGTK
LSVIPNIQNPDPAVYQLRDSKSSDKSVCLFTDFDSQTNVSQSKDSDVYITDKCVL
DMRSMDFKSNSAVAWSNKSDFACANAFNNSIIPEDT;
 und
(ii) eine Beta-Kette mit der folgenden Sequenz: 
AIQMTQSPSSLSASVGDRVTITCRASQDIRNYLNWYQQKPGKAPKLLIYYTSRLE
SGVPSRFSGSGSGTDYTLTISSLQPEDFATYYCQQGNTLPWTFGQGTKVEIKGGG
GSGGGGSGGGGSGGGGSGGGSEVQLVESGGGLVQPGGSLRLSCAASGYSFTGYTM
NWVRQAPGKGLEWVALINPYKGVSTYNQKFKDRFTISVDKSKNTAYLQMNSLRAE
DTAVYYCARSGYYGDSDWYFDVWGQGTLVTVSSGGGGSDGGITQSPKYLFRKEGQ
NVTLSCEQNLNHDAMYWYRQDPGQGLRLIYYSQIMGDEQKGDIAEGYSVSREKKE
SFPLTVTSAQKNPTAFYLCASSWWTGGASPIRFGPGTRLTVTEDLKNVFPPEVAV
FEPSEAEISHTQKATLVCLATGFYPDHVELSWWVNGKEVHSGVCTDPQPLKEQPA
LNDSRYALSSRLRVSATFWQDPRNHFRCQVQFYGLSENDEWTQDRAKPVTQIVSA
EAWGRAD;
oder
(c) das bispezifische TCR-scFv-Protein Folgendes aufweist:
(i) eine Alpha-Kette mit der folgenden Sequenz: 
GDAKTTQPNSMESNEEEPVHLPCNHSTISGTDYIHWYRQLPSQGPEYVIHGLTSN
VNNRMASLAIAEDRKSSTLILHRATLRDAAVYYCILILGHSRLGNYIATFGKGTK
LSVIPNIQNPDPAVYQLRDSKSSDKSVCLFTDFDSQTNVSQSKDSDVYITDKCVL
DMRSMDFKSNSAVAWSNKSDFACANAFNNSIIPEDT;
 und
(ii) eine Beta-Kette mit der folgenden Sequenz: 
AIQMTQSPSSLSASVGDRVTITCRASQDIRNYLNWYQQKPGKAPKLLIYYTSRLE
SGVPSRFSGSGSGTDYTLTISSLQPEDFATYYCQQGNTLPWTFGQGTKVEIKGGG
GSGGGGSGGGGSGGGGSGGGSEVQLVESGGGLVQPGGSLRLSCAASGYSFTGYTM
NWVRQAPGKGLEWVALINPYKGVSTYNQKFKDRFTISVDKSKNTAYLQMNSLRAE
DTAVYYCARSGYYGDSDWYFDVWGQGTLVTVSSGGGGSDGGITQSPKYLFRKEGQ
NVTLSCEQNLNHDAMYWYRQDPGQGLRLIYYSQIMGDEQKGDIAEGYSVSREKKE
SFPLTVTSAQKNPTAFYLCASSWWTGGSAPIRFGPGTRLTVTEDLKNVFPPEVAV
FEPSEAEISHTQKATLVCLATGFYPDHVELSWWVNGKEVHSGVCTDPQPLKEQPA
LNDSRYALSSRLRVSATFWQDPRNHFRCQVQFYGLSENDEWTQDRAKPVTQIVSA
EAWGRAD,
vorzugsweise wobei das bispezifische TCR-scFv-Protein die in Teil (b) definierten Alpha-Ketten- und Beta-Kettensequenzen aufweist.

12. Formulierung nach einem der Ansprüche 1 bis 10, wobei das bispezifische Protein eine Alpha-Kette mit der folgenden Sequenz: 
AQQGEEDPQALSIQEGENATMNCSYKTSINNLQWYRQNSGRGLVHLILIRSNERE
KHSGRLRVTLDTSKKSSSLLITASRAADTASYFCATDGSTPMQFGKGTRLSVIAN
 
IQKPDPAVYQLRDSKSSDKSVCLFTDFDSQTNVSQSKDSDVYITDKCVLDMRSMD
FKSNSAVAWSNKSDFACANAFNNSIIPEDT
 und eine Beta-Kette mit der folgenden Sequenz aufweist:

AIQMTQSPSSLSASVGDRVTITCRASQDIRNYLNWYQQKPGKAPKLLIYYTSRLE
SGVPSRFSGSGSGTDYTLTISSLQPEDFATYYCQQGNTLPWTFGQGTKVEIKGGG
GSGGGGSGGGGSGGGGSGGGSEVQLVESGGGLVQPGGSLRLSCAASGYSFTGYTM
NWVRQAPGKGLEWVALINPYKGVSTYNQKFKDRFTISVDKSKNTAYLQMNSLRAE
DTAVYYCARSGYYGDSDWYFDVWGQGTLVTVSSGGGGSDGGITQSPKYLFRKEGQ
NVTLSCEQNLNHDAMYWYRQDPGQGLRLIYYSWAQGDFQKGDIAEGYSVSREKKE
SFPLTVTSAQKNPTAFYLCASSWGAPYEQYFGPGTRLTVTEDLKNVFPPEVAVFE
PSEAEISHTQKATLVCLATGFYPDHVELSWWVNGKEVHSGVCTDPQPLKEQPALN
DSRYALSSRLRVSATFWQDPRNHFRCQVQFYGLSENDEWTQDRAKPVTQIVSAEA
WGRAD

13. Formulierung nach einem vorhergehenden Anspruch, umfassend:
50 mM Phosphat-Citrat-Puffer,
5% Trehalose,
1% Mannit und
0,02 % (w/v) Polysorbat 20,
wobei die Formulierung einen pH-Wert von 6,0, 6,5 oder 6,8 aufweist und das bispezifische Protein in einer Konzentration von 0,2 mg/ml vorliegt.

14. Formulierung nach einem der Ansprüche 1 bis 12, umfassend
20 mM Phosphat-Citrat-Puffer,
5% Trehalose,
1% Mannit und
0,02 % (w/v) Polysorbat 20,
wobei die Formulierung einen pH-Wert von 6,0, 6,5 oder 6,8 aufweist und das bispezifische Protein in einer Konzentration von 0,2 mg/ml vorliegt.

15. Formulierung nach einem vorhergehenden Anspruch, wobei die Formulierung wässrig oder lyophilisiert ist.

16. Formulierung nach einem vorhergehenden Anspruch zur medizinischen Verwendung.

17. Formulierung zur Verwendung nach Anspruch 16, zur Verwendung bei einem Verfahren zum Behandeln von Krebs.

18. Formulierung zur Verwendung nach Anspruch 17, wobei es sich bei dem Krebs um ein Melanom, vorzugsweise ein Aderhautmelanom, handelt.

## Revendications

1. Formulation pharmaceutique comprenant :
une quantité thérapeutiquement efficace d'une protéine bispécifique comprenant un récepteur de cellules T soluble (TCR) et un scFV ;
un tampon ;
un tensioactif ;
un agent de charge ; et
un stabilisant,
dans laquelle le rapport p/p du tensioactif sur la protéine se situe dans la plage allant de 0,75:1 à 1,5:1 et le rapport du stabilisant sur l'agent de charge est supérieur à 1:1, et
dans laquelle le tensioactif est un polysorbate, l'agent de charge est un polyol et le stabilisant est un disaccharide.

2. Formulation selon la revendication 1, dans laquelle le rapport p/p du tensioactif sur la protéine est de 1:1 ; et/ou
le rapport du stabilisant sur l'agent de charge est supérieur à 1,5:1, éventuellement, le rapport du stabilisant sur l'agent de charge est dans la plage de 3:1 à 7:1.

3. Formulation de la revendication 1 ou la revendication 2, dans laquelle le tensioactif est présent à une concentration en p/v de 0,1 % ou moins, éventuellement dans laquelle le tensioactif est présent à une concentration en p/v de 0,02 %.

4. Formulation selon l'une quelconque des revendications précédentes, dans laquelle le polysorbate est le polysorbate 20.

5. Formulation selon l'une quelconque des revendications précédentes, dans laquelle la concentration en p/v de l'agent de charge est comprise entre 0,9 % et 1,1 %, éventuellement dans laquelle la concentration en p/v de l'agent de charge est de 1 %.

6. Formulation selon l'une quelconque des revendications précédentes, dans laquelle le polyol est le mannitol.

7. Formulation selon l'une quelconque des revendications précédentes, dans laquelle la concentration en p/v de stabilisant se situe dans la plage de 4,5 % à 5,5 %, éventuellement, dans laquelle la concentration en p/v de stabilisant est de 5 %, et/ou le stabilisant est le saccharose ou le tréhalose, ou un mélange de tréhalose et de saccharose.

8. Formulation selon l'une quelconque des revendications précédentes, dans laquelle
la concentration de tampon est de 20 mM à 50 mM, éventuellement dans laquelle la concentration de tampon est de 50 mM, et/ou
le tampon est phosphate/citrate, et/ou
la formulation possède un pH dans la plage de 6 à 7, éventuellement dans laquelle le pH est de 6,0, 6,5 ou 6,8.

9. Formulation selon l'une quelconque des revendications précédentes, dans laquelle la concentration de la protéine bispécifique TCR-scFv est dans la plage de 0,05 à 0,5 mg/mL, éventuellement dans laquelle la concentration de la protéine bispécifique TCR-scFv est de 0,2 mg/mL.

10. Formulation selon l'une quelconque des revendications précédentes, dans laquelle
le TCR soluble reconnaît un antigène qui est présenté sur des cellules malades, l'antigène étant éventuellement présenté sur des cellules cancéreuses, des cellules infectées par des virus ou des cellules infectées par des bactéries, et/ou
le TCR soluble contient des mutations par rapport à un TCR natif ou de type sauvage, de sorte qu'il possède une affinité supra-physiologique pour un antigène, éventuellement dans laquelle l'antigène est PRAME.

11. Formulation selon l'une quelconque des revendications précédentes, dans laquelle
(a) la protéine bispécifique TCR-scFv a :
(i) une chaîne alpha ayant la séquence suivante : 
GDAKTTQPNSMESNEEEPVHLPCNHSTISGTDYIHWYRQLPSQGPEYVIHGLTSN
VNNRMASLAIAEDRKSSTLILHRATLRDAAVYYCILILGHSRAGNYIATFGKGTK
LSVIPNIQNPDPAVYQLRDSKSSDKSVCLFTDFDSQTNVSQSKDSDVYITDKCVL
DMRSMDFKSNSAVAWSNKSDFACANAFNNSIIPEDT ;
 et
(ii) une chaîne bêta ayant la séquence suivante : 
AIQMTQSPSSLSASVGDRVTITCRASQDIRNYLNWYQQKPGKAPKLLIYYTSRLE
SGVPSRFSGSGSGTDYTLTISSLQPEDFATYYCQQGNTLPWTFGQGTKVEIKGGG
GSGGGGSGGGGSGGGGSGGGSEVQLVESGGGLVQPGGSLRLSCAASGYSFTGYTM
NWVRQAPGKGLEWVALINPYKGVSTYNQKFKDRFTISVDKSKNTAYLQMNSLRAE
DTAVYYCARSGYYGDSDWYFDVWGQGTLVTVSSGGGGSDGGITQSPKYLFRKEGQ
NVTLSCEQNLNHDAMYWYRQDPGQGLRLIYYSQIMGDEQKGDIAEGYSVSREKKE
SFPLTVTSAQKNPTAFYLCASSWWTGGASPISFGPGTRLTVTEDLKNVFPPEVAV
FEPSEAEISHTQKATLVCLATGFYPDHVELSWWVNGKEVHSGVCTDPQPLKEQPA
LNDSRYALSSRLRVSATFWQDPRNHFRCQVQFYGLSENDEWTQDRAKPVTQIVSA
EAWGRAD ;
ou
(b) la protéine bispécifique TCR-scFv a :
(i) une chaîne alpha ayant la séquence suivante : 
GDAKTTQPNSMESNEEEPVHLPCNHSTISGTDYIHWYRQLPSQGPEYVIHGLTSN
VNNRMASLAIAEDRKSSTLILHRATLRDAAVYYCILILGHSRLGNYIATFGKGTK
 
LSVIPNIQNPDPAVYQLRDSKSSDKSVCLFTDFDSQTNVSQSKDSDVYITDKCVL
DMRSMDFKSNSAVAWSNKSDFACANAFNNSIIPEDT ;
 et
(ii) une chaîne bêta ayant la séquence suivante : 
AIQMTQSPSSLSASVGDRVTITCRASQDIRNYLNWYQQKPGKAPKLLIYYTSRLE
SGVPSRFSGSGSGTDYTLTISSLQPEDFATYYCQQGNTLPWTFGQGTKVEIKGGG
GSGGGGSGGGGSGGGGSGGGSEVQLVESGGGLVQPGGSLRLSCAASGYSFTGYTM
NWVRQAPGKGLEWVALINPYKGVSTYNQKFKDRFTISVDKSKNTAYLQMNSLRAE
DTAVYYCARSGYYGDSDWYFDVWGQGTLVTVSSGGGGSDGGITQSPKYLFRKEGQ
NVTLSCEQNLNHDAMYWYRQDPGQGLRLIYYSQIMGDEQKGDIAEGYSVSREKKE
SFPLTVTSAQKNPTAFYLCASSWWTGGASPIRFGPGTRLTVTEDLKNVFPPEVAV
FEPSEAEISHTQKATLVCLATGFYPDHVELSWWVNGKEVHSGVCTDPQPLKEQPA
LNDSRYALSSRLRVSATFWQDPRNHFRCQVQFYGLSENDEWTQDRAKPVTQIVSA
EAWGRAD ;
 ou
(c) la protéine bispécifique TCR-scFv a :
(i) une chaîne alpha ayant la séquence suivante : 
GDAKTTQPNSMESNEEEPVHLPCNHSTISGTDYIHWYRQLPSQGPEYVIHGLTSN
VNNRMASLAIAEDRKSSTLILHRATLRDAAVYYCILILGHSRLGNYIATFGKGTK
LSVIPNIQNPDPAVYQLRDSKSSDKSVCLFTDFDSQTNVSQSKDSDVYITDKCVL
DMRSMDFKSNSAVAWSNKSDFACANAFNNSIIPEDT ;
 et
(ii) une chaîne bêta ayant la séquence suivante : 
AIQMTQSPSSLSASVGDRVTITCRASQDIRNYLNWYQQKPGKAPKLLIYYTSRLE
SGVPSRFSGSGSGTDYTLTISSLQPEDFATYYCQQGNTLPWTFGQGTKVEIKGGG
GSGGGGSGGGGSGGGGSGGGSEVQLVESGGGLVQPGGSLRLSCAASGYSFTGYTM
NWVRQAPGKGLEWVALINPYKGVSTYNQKFKDRFTISVDKSKNTAYLQMNSLRAE
DTAVYYCARSGYYGDSDWYFDVWGQGTLVTVSSGGGGSDGGITQSPKYLFRKEGQ
NVTLSCEQNLNHDAMYWYRQDPGQGLRLIYYSQIMGDEQKGDIAEGYSVSREKKE
SFPLTVTSAQKNPTAFYLCASSWWTGGSAPIRFGPGTRLTVTEDLKNVFPPEVAV
FEPSEAEISHTQKATLVCLATGFYPDHVELSWWVNGKEVHSGVCTDPQPLKEQPA
LNDSRYALSSRLRVSATFWQDPRNHFRCQVQFYGLSENDEWTQDRAKPVTQIVSA
EAWGRAD,
de préférence dans laquelle la protéine bispécifique TCR-scFv a les séquences de chaîne alpha et de chaîne bêta définies dans la partie (b).

12. Formulation selon l'une quelconque des revendications 1 à 10, dans laquelle la protéine bispécifique a une chaîne alpha ayant la séquence suivante : 
AQQGEEDPQALSIQEGENATMNCSYKTSINNLQWYRQNSGRGLVHLILIRSNERE
KHSGRLRVTLDTSKKSSSLLITASRAADTASYFCATDGSTPMQFGKGTRLSVIAN
IQKPDPAVYQLRDSKSSDKSVCLFTDFDSQTNVSQSKDSDVYITDKCVLDMRSMD
FKSNSAVAWSNKSDFACANAFNNSIIPEDT
 et une chaîne bêta ayant la séquence suivante :

AIQMTQSPSSLSASVGDRVTITCRASQDIRNYLNWYQQKPGKAPKLLIYYTSRLE
SGVPSRFSGSGSGTDYTLTISSLQPEDFATYYCQQGNTLPWTFGQGTKVEIKGGG
GSGGGGSGGGGSGGGGSGGGSEVQLVESGGGLVQPGGSLRLSCAASGYSFTGYTM
NWVRQAPGKGLEWVALINPYKGVSTYNQKFKDRFTISVDKSKNTAYLQMNSLRAE
DTAVYYCARSGYYGDSDWYFDVWGQGTLVTVSSGGGGSDGGITQSPKYLFRKEGQ
NVTLSCEQNLNHDAMYWYRQDPGQGLRLIYYSWAQGDFQKGDIAEGYSVSREKKE
SFPLTVTSAQKNPTAFYLCASSWGAPYEQYFGPGTRLTVTEDLKNVFPPEVAVFE
PSEAEISHTQKATLVCLATGFYPDHVELSWWVNGKEVHSGVCTDPQPLKEQPALN
DSRYALSSRLRVSATFWQDPRNHFRCQVQFYGLSENDEWTQDRAKPVTQIVSAEA
WGRAD.

13. Formulation selon l'une quelconque des revendications précédentes, comprenant
un tampon phosphate-citrate 50 mM,
5 % de tréhalose,
1 % de mannitol, et
0,02 % p/v de polysorbate 20,
la formulation ayant un pH de 6,0, 6,5 ou 6,8 et la protéine bispécifique étant présente à une concentration de 0,2 mg/mL.

14. Formulation selon l'une quelconque des revendications 1 à 12, comprenant
un tampon phosphate-citrate 20 mM,
5 % de tréhalose,
1 % de mannitol, et
0,02 % p/v de polysorbate 20,
la formulation ayant un pH de 6,0, 6,5 ou 6,8 et la protéine bispécifique étant présente à une concentration de 0,2 mg/mL.

15. Formulation selon l'une quelconque des revendications précédentes, dans laquelle la formulation est aqueuse ou lyophilisée.

16. Formulation selon l'une quelconque des revendications précédentes pour une utilisation en médecine.

17. Formulation pour une utilisation selon la revendication 16, pour une utilisation dans un procédé pour le traitement d'un cancer.

18. Formulation pour une utilisation selon la revendication 17, dans laquelle le cancer est un mélanome, de préférence un mélanome uvéal.
